# EUROPÄISCHE PATENTSCHRIFT

(11) **EP 1 162 941 B1**
(45) Veröffentlichungstag und Bekanntmachung des Hinweises auf die Patenterteilung: **24.05.2006**
(21) Anmeldenummer: 00909299.0
(22) Anmeldetag: 03.03.2000
(51) Int. Cl.: A61K 8/73, A61Q 17/00

(54) **SONNENSCHUTZMITTEL**
SUN PROTECTION AGENTS
AGENTS DE PROTECTION CONTRE LE SOLEIL

(30) Priorität: 12.03.1999 DE 19911052
(43) Veröffentlichungstag der Anmeldung: 19.12.2001
(73) Patentinhaber: Biotec Pharmacon ASA, 9008 Tromsö (NO)
(72) Erfinder: GRIESBACH, Ute, D-40597 Düsseldorf (DE); WACHTER, Rolf, D-40595 Düsseldorf (DE); FABRY, Bernd, D-41352 Korschenbroich (DE); HEYER, Michael, D-40699 Erkrath (DE); ENGSTAD, Rolf, E., Biotec ASA, N-9008 Tromso (NO)
(74) Vertreter: Melzer, Wolfgang
(86) Internationale Anmeldenummer: PCT/EP2000/001835
(87) Internationale Veröffentlichungsnummer: WO 2000/054734

(56) Entgegenhaltungen:
- EP-A- 0 681 830
- EP-A- 0 875 244
- WO-A-95/30022
- US-A- 5 397 773

## Beschreibung

### Gebiet der Erfindung

Die Erfindung befindet sich auf dem Gebiet der Sonnenkosmetik und betrifft neue Zubereitungen mit einem Gehalt an speziellen Glucanen und UV-Lichtschutzfaktoren sowie die Verwendung der Glucane zur Verbesserung der Photostabilität der Lichtschutzfaktoren.

### Stand der Technik

Die kosmetischen Sonnenschutzprodukte schützen die menschliche Haut nicht nur vor den direkten Folgen der UV-A und UV-B-Strahlung, also vor Sonnenbrand, sondern auch vor den indirekten Folgen in Form von Hautalterung und Hautkrebs. Deshalb werden in der Regel organische Filtersubstanzen eingesetzt, die aufgrund ihrer chemischen Struktur in der Lage sind, ultraviolettes Licht zu absorbieren. Durch die Aufnahme der UV-Strahlung werden im Filtermolekül Elektronen in einen angeregten Zustand versetzt. Die dabei aufgenommene Energie wird anschließend in Form von Wärmestrahlung wieder freigesetzt, wobei photostabile Moleküle in ihren energetischen Grundzustand zurückkehren, während bei photoinstabilen Filtern, zu denen ein Großteil der bekannten Lichtschutzfaktoren zählt, eine chemische Veränderung eintritt, wie beispielsweise eine Isomerisierung oder ein radikalischer Abbau. Neben der nicht immer befriedigenden Photostabilität der Lichtschutzfaktoren besteht ein weiteres Problem darin, daß die Filter - zumal in kosmetischen Ölen - nur eine begrenzte Löslichkeit bzw. Dispergierbarkeit besitzen. Hierdurch kann es geschehen, daß die Stoffe im Laufe der Lagerung ausfallen bzw. sedimentieren, wodurch das Lichtschutzpräparat einen wesentlichen Teil seiner Leistung einbüßt.

In diesem Zusammenhang sei auf die Europäische Patentschrift **EP-B1 0500718** (Donzis) verwiesen, aus der die Verwendung von Glucanen in Sonnenschutrmittefn bekannt ist Die Glucane werden durch Extraktion aus den Zellwänden von Hefen gewonnen, sind wasserunlöslich und enthalten (1,6)-Ver-knüpfungen. Des weiteren werden in der **EP-A1 0681830** (Unilever) Sonnenschutzmittel vorgeschlagen, die Ethylen/Vinylacetat-Copolymere und Polyacrylate, UV-Lichtschutzfifter und gegebenenfalls noch bis zu 10 Gew.% Glucane enthalten Demzufolge hat die Aufgabe der vorliegenden Erfindung darin bestanden, sowohl die Öllöslichkeit bzw. Öldispergierbarkeit von UV-Lichtschutzfaktoren als auch deren Photostabilität zu verbessern. Des weiteren sollten Zubereitungen zur Verfügung gestellt werden, die sich gegenüber den Produkten des Stands der Technik durch immunmodulierende bzw. immunstimulierende Eigenschaften, verbesserte Haftung, Filmbildung und Wasserbeständigkeit auszeichnen sollten.

### Beschreibung der Erfindung

Gegenstand der Erfindung sind Sonnenschutzmittel, enthaltend
(a) wasserlösliche β-(1,3)-Glucane, im wesentlichen frei von β-(1,6)-Verknüpfungen, deren Seitenketten (1,3)-Verknüpfungen aufweisen und
(b) UV-Lichtschutzfaktoren.

Überraschenderweise wurde gefunden, daß der Zusatz von β-(1,3)-Glucanen, die im wesentlichen frei von β-(1,6)-Verknüpfungen sind nicht nur die Löslickeit bzw. Dispergierbarkeit von UV-Lichtschutz-faktoren ir kosmetischen Ölen steigert, sondern auch deren Photostabilität entscheidend verbessert, während Glucane, zumal wasserunlöslich, die noch über nennenswerte Gehalte an β-(1,6)-Ver-knüpfungen verfügen, weder in Richtung Solubilisierung noch in Richtung Photostabilität einen Effekt zeigen. Die Erfindung schließt die Erkenntnis ein, daß die Mittel das Immunsystem anregen sowie über eine verbesserte Haftung, Filmbildung und Wasserbeständigkeit verfügen.

### Wasserlösliche β-(1,3)-Glucane

Unter der Bezeichnung Glucane werden Homopolysaccharide auf Basis der Glucose verstanden. Je nach sterischer Verknüpfung unterscheidet man zwischen β-(1,3)-, β-(1,4)- und β-(1,6)-Glucanen. β-(1,3)-Glucane weisen meist eine helicale Struktur auf, während Glucane mit einer 1,4-Verknüpfung im allgemeinen eine lineare Struktur besitzen. Die β-Glucane der Erfindung besitzen eine (1,3)-Struktur, d.h. sie sind weitgehend frei von unerwünschten (1,6)-Verknüpfungen. Vorzugsweise werden solche β -(1,3)-Glucane eingesetzt, deren Seitenketten (1,3)-Verknüpfungen aufweisen. Insbesondere enthalten die Mittel Glucane, die auf Basis von Hefen der Familie *Saccharomyces,* speziell *Saccharomyces cerevisiae* erhalten werden. Glucane dieser Art sind in technischem Maße nach bekannten Verfahren zugänglich. So beschreibt z. B.die Internationale Patentanmeldung **WO 95/30022** (Biotec-Mackzymal) ein Verfahren zur Herstellung solcher Stoffe, bei dem man Glucane mit β-(1,3)- und β-(1,6)-Verknüpfungen in solcher Weise mit β-(1,6)-Glucanasen in Kontakt bringt, daß praktisch alle β-(1,6)-Verknüpfüngen gelöst werden. Vorzugsweise werden zur Herstellung dieser Glucane Glucanasen auf Basis von *Trichodermia harzianum* eingesetzt. Soweit es die Herstellung und Zugänglichkeit der in den erfindungsgemäßen Mitteln enthaltenen Glucane angeht, wird in vollem Umfang auf die Offenbarung der oben genannten Schrift Bezug genommen.

### UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahtung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimisäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthyiester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester,
- Triazinderivate, wie z.B. 2,4,6-Trianilino-(p-carbo-2'-ethyl-1'-hexyloxy)-1,3,5-triazin und Octyl Triazon, wie in der **EP-A1 0818450** beschrieben;
- Propan-1,3-dione, wie zB. 1-(4-tert.Butylphenyl)-3-(4'methoxyphenyl)propan-1,3-dion;
- Ketotricyclo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Hierbei zeichnen sich Kombinationen von Octocrylene bzw. Campherderivaten mit Butyl Methoxydibenzoylmethane durch besondere Photostabitität aus.

Neben den genannten löslichen Stoffen kommen für diesen Zweck auch unlösliche Lichtschutz-pigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. In Sonnenschutzmitteln werden bevorzugt sogenannte Mikro- oder Nanopigmente eingesetzt. Vorzugsweise wird mikronisiertes Zinkoxid verwendet.

Weitere geeignete UV-Lichtschutzfaktoren sind der Übersicht von P.Finkel in **SÖFW-Journal 122, 543 (1996)** zu entnehmen. Ebenfalls geeignet sind pflanzliche Extrakte mit UV-absorbierenden bzw. antioxidativen Eigenschaften. In einer bevorzugten Ausführungsform der Erfindung enthalten die Mittel
(a) 0,01 bis 25, vorzugsweise 0,5 bis 15 und insbesondere 1 bis 5 Gew.-% wasserlösliche β-(1,3)-Glucane, die im wesentlichen frei von β-(1,6)-Verknüpfungen sind, deren Seitenketten (1,3)-Verknüpfungen aufweisen und
(b) 0,05 bis 10, vorzugsweise 0,5 bis 8 und insbesondere 1 bis 5 Gew.-% UV-Lichtschutzfaktoren,
mit der Maßgabe, daß sich die Mengenangaben mit Wasser sowie gegebenenfalls weiteren Hilfs- und Zusatzstoffen zu 100 Gew.% ergänzen.

### Gewerbliche Anwendbarkeit

Der Zusatz von β-(1,3)-Glucanen, die im wesentlichen frei von β-(1,6)-Verknüpfungen sind, verbessert nicht nur die Löslichkeit bzw. Dispergierbarkeit von UV-Lichtschutzfaktoren in kosmetischen Ölen, sondern steigert zudem auch ihre Photostabilität. Ein weiterer Gegenstand der Erfindung betrifft daher die Verwendung der genannten Stoffe zur Verbesserung der Photostabilität und Löslichkeit von UV-Lichtschutzfaktoren in Sonnenschutzmitteln.

### Sonnenschutzmittel

Die erfindungsgemäßen Sonnenschutzmittel, wie beispielsweise Cremes, Lotionen, Öle, Gele, Sticks und dergleichen, können ferner als weitere Hilfs- und Zusatzstoffe milde Tenside, Ölkörper, Emulgatoren, Überfettungsmittel, Perlglanzwachse, Konsistenzgeber, Verdickungsmittel, Polymere, Siliconverbindungen, Fette, Wachse, Stabilisatoren, biogene Wirkstoffe, Deowirkstoffe, Antischuppenmittel, Filmbildner, Quell-mittel, Antioxidantien, anorganische Farbpigmente, Hydrotrope, Konservierungsmittel, Insektenrepellentien, Selbstbräuner, Solubilisatoren, Parfümöle, Farbstoffe und dergleichen enthalten.

Typische Beispiele für geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestem mit Polyolen, Siliconöle und/oder aliphatische bzw. naphthenische Kohlenwasserstoffe, wie z.B. wie Squalan, Squalen oder Dialkylcyclohexane in Betracht.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimeratisostearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zukkeralkohole (z.B. Sorbit), Alkylglucoside (z.B. Methylgtucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18-}Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquatemierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycotester, speziell Ethylengfycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten.

Geeignete **Verdickungsmittel** sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthafene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quatemierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchtorid (Merquat® 550/Chemviron), Polyaminopolyamide, wie z.B. beschrieben in der **FR 2252840 A** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vnylacetat/Crotonsäure-Copolymere, Vinylpyrrolidon/Vinylacrylat-Copolymere, Vnylacetat/Butylmaleat/Isobornylacrylat-Copolymere, Methylvinylether/Mateinsäureanhydrid-Copolymere und deren Ester, unvemetzte und mit Polyolen vemetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen. Kettenlänge von 200 bis 300 Dimethylsiloxan-Einheiten und hydrierten Silicaten händelt. Eine detaillierte Übersicht über geeignete flüchtige Silicone findet sich zudem von Todd et al. in **Cosm.Toil. 91, 27 (1976).**

Typische Beispiele für Fette sind Glyceride, als Wachse kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie zB. Montanesterwachse, Sasolwachse, hydrierte Jojobawachse sowie synthetische Wachse, wie z.B. Polyalkylenwachse und Polyethylenglycolwachse in Frage.

Als Stabilisatoren können Metallsalze von Fettsäuren, wie z.B. Magnesium-, Aluminium- und/oder Zinkstearat bzw. -ricinoleat eingesetzt werden.

Unter biogenen **Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen.

Als **Deowirkstoffe** kommen z.B. Antiperspirantien wie etwa Aluminiumchlorhydrate in Frage. Hierbei handelt es sich um farblose, hygroskopische Kristalle, die an der Luft leicht zerfließen und beim Eindampfen wäßriger Aluminiumchloridlösungen anfallen. Aluminiumchlorhydrat wird zur Herstellung von schweißhemmenden und desodorierenden Zubereitungen eingesetzt und wirkt wahrscheinlich über den partiellen Verschluß der Schweißdrüsen durch Eweiß- und/oder Polysaccharidfällung [vgl. **J.Soc. Cosm.Chem. 24, 281 (1973)].** Unter der Marke Locron® der Hoechst AG, Frankfurt/FRG, befindet beispielsweise sich ein Aluminiumchlorhydrat im Handel, das der Formel [Al₂(OH)₅Cl]*2,5 H₂O entspricht und dessen Einsatz besonders bevorzugt ist [vgl. **J.Pharm.Pharmacol. 26, 531 (1975)].** Neben den Chlorhydraten können auch Aluminiumhydroxylactate sowie saure Aluminium/Zirkoniumsalze eingesetzt werden. Als weitere Deowirkstoffe können Esteraseinhibitoren zugesetzt werden. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT, Henkel KGaA, Düsseldorf/FRG). Die Stoffe inhibieren die Enzymaktivität und reduzieren dadurch die Geruchsbildung. Wahrscheinlich wird dabei durch die Spaltung des Citronensäureesters die freie Säure freigesetzt, die den pH-Wert auf der Haut soweit absenkt, daß dadurch die Enzyme inhibiert werden. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester. Antibakterielle Wirkstoffe, die die Keimflora beeinflussen und schweißzersetzende Bakterien abtöten bzw. in ihrem Wachstum hemmen, können ebenfalls in den Stiftzubereitungen enthalten sein. Beispiele hierfür sind Chitosan, Phenoxyethanol und Chlorhexidingluconat. Besonders wirkungsvoll hat sich auch 5-Chlor-2-(2,4-dichlorphen-oxy)-phenol erwiesen, das unter der Marke Irgasan® von der Ciba-Geigy, Basel/CH, vertrieben wird.

Als **Antischuppenmittel** können Climbazol, Octopirox und Zinkpyrethion eingesetzt werden. Gebräuchliche **Filmbitdner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pernulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

Neben den primären Lichtschutzstoffen können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Camosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze. Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascor bylphosphat, Ascorbylacetat), Tocopherole und Derivate (z.B. Vitamin-E-acetat), Vitamin A und Derivate (Vitamin-A-palmitat) sowie Kaniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise. Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2 Amino-1,3-propandiol.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Insekten-Repellent 3535 in Frage, als Selbstbräuner eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedem-, Rosenholz), Kräutem und Gräsem (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Typische Beispiele für **keimhemmende Mittel** sind Konservierungsmittel mit spezifischer Wirkung gegen gram-positive Bakterien wie etwa 2,4,4'-Trichlor-2'-hydroxydiphenylether, Chlorhexidin (1,6-Di-(4 chlorphenyl-biguanido)-hexan) oder TCC (3,4,4'-Trichlorcarbanilid). Auch zahlreiche Riechstoffe und etherische Öle weisen antimikrobielle Eigenschaffen auf. Typische Beispiele sind die Wirkstoffe Eugenot, Menthol und Thymol in Nelken-, Minz- und Thymianöl. Ein interessantes natürliches Deomittel ist der Terpenalkohol Famesol (3,7,11-Trimethyl-2,6,10-dodecatrien-1-ol), der im Lindenblütenöl vorhanden ist und einen Maiglöckchengeruch hat. Auch Glycerinmonolaurat hat sich als Bakteriostatikum bewährt. Üblicherweise liegt der Anteil der zusätzlichen keimhemmenden Mittel bei- etwa 0,1 bis 2 Gew.-% - bezogen auf den auf den Feststoffanteil der Zubereitungen.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur Methode.

### Beispiele

Die Photostabilität verschiedener UV-A-Filter in Abmischung mit unterschiedlichen Ölkomponenten wurde nach der von der Firma Merck anläßlich des APV-Seminars vom 17.-18.9.1997 in Fulda veröffentlichten Methode bestimmt. Die Ergebnisse sind in Tabelle 1 zusammengefaßt. Zur Bestimmung der Öllöslichkeit wurde die Sättigungskonzentration verschiedener UV-Lichtschutzfaktoren in unterschiedlichen kosmetischen Ölen in Gegenwart bzw. in Abwesenheit von 0,5 Gew.-% β-(1,3)-Glucan ermittelt. Die Beispiele 1 bis 6 sind erfindungsgemäß, die Beispiele V1 und V2 dienen zum Vergleich.

**Tabelle 1 Photostabilität**

| **Zusammensetzung / Performance** | **1** | **2** | **3** | **4** | **5** | **6** | **V1** | **V2** |
|---|---|---|---|---|---|---|---|---|
| Octocrylene | 1,0 | - | - | - | - | - | 1,0 | |
| Benzophenone-3 | - | 1,0 | - | - | - | - | - | 1,0 |
| Isoamyl p-Methoxycinnamate | - | - | 1,0 | - | - | - | - | - |
| Octyl Methoxycinnamate | - | - | - | 1,0 | - | - | - | - |
| Octyl Triazone | - | - | - | - | 1,0 | - | - | - |
| 4-tert.-Butyl-4'-methoxydibenzoylmethan | - | - | - | - | - | 1,0 | - | - |
| Betaglucan* | 0,5 | 0,5 | 0,5 | 0,5 | 0,5 | 0,5 | - | - |
| Cocoglycerides | 8,5 | 8,5 | - | - | - | - | 9,0 | 9,0 |
| Dicaprylyl Ether | - | - | 8,5 | 8,5 | - | - | - | - |
| Paraffinöl | - | - | - | - | 8,5 | 8,5 | - | - |
| Wasser | ad 100 | | | | | | | |
| ***Photostabilität [%-rel.]*** | 100 | 100 | 100 | 99 | 99 | 9B | 93 | 92 |
| ***Öllöslichkeit [Gew.-%]*** | 40 | 35 | 42 | 45 | 47 | 45 | 35 | 31 |

| | | | | | | | | |
|---|---|---|---|---|---|---|---|---|
| *) Highcareen® GS (Henkel KGaA, Düsseldorf) | | | | | | | | |

Die Beispiele zeigen, daß in Gegenwart von β-(1,3)-Glucanen sowohl die Photostabilität der Lichtschutzfaktoren als auch deren Löslichkeit in kosmetischen Ölen signifikant verbessert wird. In der nachfolgenden Tabelle 2 sind eine Reihe von Formulierungsbeispielen enthalten.

**Tabelle 2 Sonnenschutzmittel (Wasser, Konservierungsmittel ad 100 Gew.-%)**

| **Zusammensetzung (INCl)** | **1** | **2** | **3** | **4** | **5** | **6** | **7** | **8** | **9** | **10** |
|---|---|---|---|---|---|---|---|---|---|---|
| **Dehymuls®** PGPH Polyglyceryl-2 Dipolyhydroxystearate | 2,0 | 3,0 | - | 5,0 | - | - | - | - | - | - |
| **Lameform® TGI** Polyglyceryl-3 Diisostearate | 4,0 | 1,0 | - | - | - | - | - | - | - | - |
| **Ambil® EM 90** Cetyl Dimethicone Copolyol | - | - | 3,0 | - | - | - | - | - | - | - |
| **Emulgade® PL 68/50** Cetearyl Glucoside (and) Cetearyl Alcohol | - | - | - | - | 4,0 | - | - | - | 3,0 | - |
| **Eumulgin®B2** Ceteareth-20 | - | - | - | - | - | - | - | 2,0 | - | - |
| **Tegocare® PS** Polyglyceryl-3 Methylglucose Distearate | - | - | - | - | - | - | 4,0 | - | - | - |
| **Eumulgin® VL 75** Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2.0 | 5,0 | 2,0 | - | - | - | - | - | - |
| **Cutina® GMS** Glyceryl Stearate | - | - | - | - | - | 2,0 | 4.0 | - | - | 4,0 |
| **Lanette® O** Cetearyl Alcohol | - | - | 2,0 | - | 2,0 | 4,0 | 2,0 | 4,0 | 4.0 | 1,0 |
| **Antaron®V216** PVP / Hexadecene Copolymer | - | - | | - | - | 3,0 | - | - | - | 2,0 |
| **Myritol®331** Cocoglycerides | 5,0 | - | 10,0 | - | 8,0 | 6,0 | 6,0 | - | 5,0 | 5,0 |
| **Finsolv®TN** C12/15 Alkyl Benzoate | - | 6,0 | - | 2,0 | - | - | 3,0 | - | - | 2,0 |
| **Cetiol® J 600** Oleyl Enrucate | 7,0 | 4,0 | 3,0 | 5,0 | 4,0 | 3,0 | 3,0 | - | 5,0 | 4,0 |
| **Cetiol® OE** Dicaprylyl Ether | 3,0 | - | 6,0 | 8,0 | 6,0 | 1,0 | 4,0 | 3,0 | 4,0 | 6,0 |
| **Mineral Oil** | - | 4.0 | - | 4,0 | - | 2,0 | - | 1,0 | - | - |
| **Cetiol® PGL** Hexadecanol (and) Hexyldecyl Laurate | - | 7,0 | 3,0 | 7,0 | 4,0 | - | - | - | 1,0 | - |
| **Panthenol/Bisaholol** | 1,2 | 1,2 | 1,2 | 1,2 | 1,2 | 1,2 | 1,2 | 1,2 | 1,2 | 1,2 |
| **Highcareen® GS** Betaglucan | 1,0 | 1,0 | 1,0 | 1,0 | 1,0 | 1,0 | 1,0 | 1,0 | 1,0 | 1,0 |
| **Copherol® F 1300** Tocopherol/Tocopheyl Acetate | 0,5 | 1,0 | 1,0 | 2,0 | 1,0 | 1,0 | 1,0 | 2,0 | 0,5 | 2,0 |
| **Neo Heliopan® Hydro** Sodium Phenylbenzimidazole Sulfonate | 3,0 | - | - | 3,0 | - | - | 2,0 | - | 2,0 | - |
| **Neo Heliopan® 303** Octocrylene | - | 5,0 | - | - | - | 4,0 | 5,0 | - | - | 10,0 |
| **Neo Heliopan® BB** Benzophenone-3 | 1,5 | . | - | 2,0 | 1,5 | - | - | - | 2,0 | |
| **Neo Heliopan® E 1000** Isoamyl-p-Methoxycinnamante | 5,0 | - | 4,0 | - | 2,0 | 2,0 | 4,0 | 10,0 | - | - |
| **Neo Heliopan® AV** Octyl Methoxycinnamate | 4,0 | - | 4,0 | 3,0 | 2.0 | 3,0 | 4,0 | - | 10,0 | 2,0 |
| **Uvinul® T 150** Octyl Triazone | 2,0 | 4,0 | 3,0 | 1,0 | 1,0 | 1,0 | 4,0 | 3,0 | 3,0 | 3,0 |
| **Zinc Oxide** | - | - | - | - | 4,0 | - | - | - | - | 5,0 |
| **Titanium Dioxide** | - | 2,0 | 2,0 | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |

| | | | | | | | | | | |
|---|---|---|---|---|---|---|---|---|---|---|
| (1) W/O-Sonnenschutzcreme, (2-4) W/O-Sonnenschutzlotion, (5, 8, 10) O/W-Sonnenschutzlotion, (6,7, 9) O/W-Sonnenschutzcreme | | | | | | | | | | |

## Patentansprüche

1. Sonnenschutzmittel, enthaltend
(a) wasserlösliche β-(1,3)-Glucane, im wesentlichen frei von β-(1,6)-Verknüpfüngen, deren Seitenketten (1,3)-Verknüpfungen aufweisen, und
(b) UV-Lichtschutzfaktoren.

2. Mittel nach Anspruch 1,
**dadurch gekennzeichnet,**
**dass** sie Glucane enthalten, die auf Basis von Hefen der Familie Saccharomyces erhalten werden.

3. Mittel nach den Ansprüchen 1 und/oder 2,
**dadurch gekennzeichnet,**
**dass** sie Glucane enthalten, die erhalten werden, indem man Glucane mit β-(1,3)- und β-(1,6)-Verknüpfungen in solcher Weise mit β-(1,6)-Glucanasen in Kontakt bringt, dass praktisch alle β-(1,6)-Verknüpfungen gelöst werden.

4. Mittel nach Anspruch 3,
**dadurch gekennzeichnet,**
**dass** man Glucane einsetzt, die zuvor mit Glucanasen auf Basis von Trichodermia harzianum behandelt worden sind.

5. Mittel nach mindestens einem der Ansprüche 1 bis 4,
**dadurch gekennzeichnet,**
**dass** sie öllösliche UVB-Lichtschutzfaktoren enthalten, die ausgewählt sind aus der Gruppe, die gebildet wird von 3-Benzylidencampher bzw. 3-Benzylidennorcampher und dessen Derivaten, 4-Aminobenzoesäurederivaten, Zimtsäureestern, Salicylsäureestern, Benzalmalonsäureestern, Benzophenonderivaten, Triazinderivaten und Propan-1,3-dionen.

6. Mittel nach mindestens einem der Ansprüche 1 bis 5,
**dadurch gekennzeichnet,**
**dass** sie wasserlösliche UVB-Lichtschutzfaktoren enthalten, die ausgewählt sind aus der Gruppe, die gebildet wird on 2-Phenylbenzimidazol-5-sulfonsäure und deren Alkali-, Erdalkali-, Ammonium-, Alkylammonium-, Alkanolammonium- und Glucanammoniumsalze sowie Sulfonsäurederivaten von Benzophenonen oder 3-Benzylidencampher.

7. Mittel nach mindestens einem der Ansprüche 1 bis 6,
**dadurch gekennzeichnet,**
**dass** sie UVA-Lichtschutzfaktoren enthalten, die ausgewählt sind aus der Gruppe, die gebildet wird von 1-(4'-tert. Butylphenyl)-3-(4'-methoxyphenyl)propan-1,3-dion, 4-tert.-Butyl-4'-methoxydibenzoylmethan und 1-Phenyl-3-(4'-isopropylphenyl)propan-1,3-dion.

8. Mittel nach mindestens einem der Ansprüche 1 bis 7,
**dadurch gekennzeichnet,**
**dass** sie unlösliche Lichtschutzpigmente enthalten, die ausgewählt sind aus der Gruppe, die gebildet wird von feindispersen Oxiden des Zinks, Titans, Eisens, Zirkoniums, Siliciums, Mangans, Aluminiums und Cers sowie feindispersen Silicaten, Bariumsulfat und Zinkstearat.

9. Mittel nach mindestens einem der Ansprüche 1 bis 8,
**dadurch gekennzeichnet, dass** sie
(a) 0,01 bis 25 Gew.-% wasserlösliche β-(1,3)-Glucane, die im wesentlichen frei von β-(1,6)-Verknüpfungen sind und deren Seitenketten (1,3)-Verknüpfungen aufweisen und
(b) 0,05 bis 10 Gew. % UV-Lichtschutzfaktoren
enthalten, mit der Maßgabe, dass sich die Mengenangaben mit Wasser sowie gegebenenfalls weiteren Hilfs- und Zusatzstoffen zu 100 Gew. % ergänzen.

10. Verwendung von wasserlöslichen β-(1,3)-Glucanen, die im wesentlichen frei von β-(1,6)-Verknüpfungen sind und deren Seitenketten β-(1,3)-Verknüpfungen aufweisen, zur Verbesserung der Photostabilität und Löslichkeit von UV-Lichtschutzfaktoren in Sonnenschutzmitteln.

## Claims

1. Sun protection agents comprising
(a) water-soluble β-(1,3)-glucans, substantially free of β-(1,6)-linkages whose side-chains comprise (1,3)-linkages, and
(b) UV light protection factors.

2. The agents according to claim 1 **characterized by** comprising glucans which are obtained based on yeasts of the family *Saccharomyces.*

3. The agents according to claim 1 and/or claim 2 **characterized by** comprising glucans which are obtained by contacting glucans with β-(1,3)- and β-(1,6)-linkages with β-(1,6)-glucanase such that practically all β-(1,6)-linkages are cleaved.

4. The agents according to claim 3 **characterized in that** glucans are used which have previously been treated with glucanases based on *Trichodermia harzianum.*

5. The agents according to at least one of claims 1 to 4 **characterized by** comprising oil-soluble UVB-light protection factors selected from the group formed by 3-benzylidene camphor and 3-benzylidene norcamphor respectively, and derivatives thereof, 4-aminobenzoic acid derivatives, cinnamon acid esters, salicylic acid esters, benzalmalonic acid esters, benzophenon derivatives, triazine derivatives and propane- 1,3-diones.

6. The agents according to at least one of claims 1 to 5 **characterized by** comprising water-soluble UVB-light protection factors selected from the group formed by 2-phenylbenzimidazol-5-sulfonic acid and its alkali, alkali earth, ammonium, alkyl ammonium, alkanol ammonium and glucammonium salts as well as sulfonic acid derivatives of benzophenones or 3-benzylidene camphor.

7. The agents according to at least one of claims 1 to 6 **characterized by** comprising UVA-light protection factors selected from the group formed by 1-(4'-tert-butylphenyl)-3-(4'-methoxyphenyl)propane-1,3-dion, 4-tert-butyl-4'-methoxy dibenzoyl methane and 1-phenyl-3-(4'-isopropylphenyl)propane-1,3-dion.

8. The agents according to at least one of claims 1 to 7 **characterized by** comprising insoluble light protection pigments selected from the group formed by fine disperse oxides of zinc, titanium, iron, zirconium, silicon, manganese, aluminium and cerium as well as fine disperse silicates, barium sulphate and zinc stearate.

9. The agents according to at least one of claims 1 to 8 **characterized by** comprising:
(a) 0.01 to 25% by weight of water-soluble β-(1,3) glucans which are substantially free of β-(1,6)-linkages and whose side-chains comprise (1,3)-linkages, and
(b) 0.05 to 10% by weight of UV light protection factors,
provided that the amounts complement with water and optional further auxiliary and additional substances to 100 % by weight.

10. Use of water-soluble β-(1,3) glucans which are substantially free of β-(1,6)-linkages and whose side-chains comprise (1,3)-linkages for improving the photo stability and solubility of UV-light protection factors in sun protection agents.

## Revendications

1. Agents de protection solaire, comprenant
a) des β-(1,3)-glucanes solubles dans l'eau, essentiellement libérés de liaisons β-(1,6), dont les chaînes latérales comportent des liaisons (1,3) et
b) des facteurs de protection contre les rayons UV

2. Agents selon la revendication 1, **caractérisés en ce qu'**ils contiennent des glucanes qui sont obtenus à partir de levures de la famille des Saccharomyces.

3. Agents selon revendications 1 et/ou 2, **caractérisés en ce qu'**ils contiennent des glucanes qui sont obtenus par le fait qu'on mette en contact des glucanes à liaisons β-(1,3)- et β-(1,6) à des β-(1,6)-glucanases de telle façon que pratiquement toutes les liaisons β-(1,6)- soient clivées.

4. Agents selon la revendication 3, **caractérisés en ce qu'**on introduise des glucanes qui ont été traités au préalable avec des glucanases provenant du Trichodermia harzianum.

5. Agents selon au moins l'une des revendications 1 à 4, **caractérisés en ce qu'**ils contiennent des facteurs oléosolubles de protection contre les rayons UVB sélectionnés parmi le groupe composé du 3-benzylidène camphre ou plutôt du 3-benzylidène norcamphre et leurs dérivés, des dérivés d'acide 4-aminobenzoïque, des esters d'acide cinnamique, des esters d'acide salicylique, des esters d'acide benzal malonique, des dérivés de benzophénone, des dérivés de triazine et du propane-1,3-dione.

6. Agents selon au moins une des revendications 1 à 5, **caractérisés en ce que** lesdits agents contiennent des facteurs de protection contre les rayons UVB solubles dans l'eau sélectionnés parmi le groupe composé de l'acide 2-phenylbenzimidazol-5-sulfonique et ses sels alcali, alcalino-terreux, ammonium, alkylammonium, alkanol ammonium et glucanammonium aussi bien que les dérivés d'acide sulfonique de benzophénone ou du 3-benzylidène camphre.

7. Agents selon au moins une des revendications 1 à 6, **caractérisé en ce que** lesdits agents contiennent des facteurs de protection contre les rayons UVA sélectionnés parmi le groupe composé de 1-(4'-tert. Butylphenyl)-3-(4'-methoxyphenyl)-propan-1,3-dione, 4-tert.-Butyl-4'-methoxydibenzoylméthane et 1-Phényl-3-(4'-isopropylphényl)propan-1,3-dione.

8. Agents selon au moins une des revendications 1 à 7, **caractérisé en ce que** lesdits agents contiennent des pigments insolubles pour la protection contre la lumière, sélectionnés parmi le groupe composé d'oxydes de zinc, de titane, de fer, de zirconium, de silicium, de manganèse, d'aluminium, de cérium finement dispersés ainsi que des silicates, du sulfate de baryum et du stéarate de zinc.

9. Agents selon au moins une des revendications 1 à 8, **caractérisé en ce que** lesdits agents comprennent
a. 0.01 à 25 % par poids de β-(1,3)-glucanes solubles dans l'eau, qui sont essentiellement libérés de liaisons β-(1,6) et dont les chaînes latérales comportent des liaisons (1,3)- et
b. 0.05 à 10 % par poids de facteurs de protection contre les rayons UV
pour autant que les quantités données soient complétées jusqu'à 100% par poids avec de l'eau et si nécessaire, avec d'autres excipients et additifs.

10. Utilisation des β-(1,3)-glucanes solubles dans l'eau qui sont essentiellement libérés des liaisons β-(1,6) et dont les chaînes latérales comportent des liaisons β-(1,3) pour l'amélioration de la photostabilité et de la solubilité des facteurs de protection contre les rayons UV dans les agents de protection solaire.
